# EUROPÄISCHE PATENTSCHRIFT

(11) **EP 0 810 029 B1**
(45) Veröffentlichungstag und Bekanntmachung des Hinweises auf die Patenterteilung: **14.11.2001**
(21) Anmeldenummer: 97107505.6
(22) Anmeldetag: 07.05.1997
(51) Int. Cl.: B01J 31/22, C07C 45/50

(54) **Verfahren zur Herstellung von Aldehyden**
Aldehydes preparation process
Procédé de préparation d'aldéhydes

(30) Priorität: 13.08.1996 DE 19632602
(43) Veröffentlichungstag der Anmeldung: 03.12.1997
(73) Patentinhaber: Celanese Chemicals Europe GmbH, 60439 Frankfurt am Main (DE)
(72) Erfinder: Bahrmann, Helmut, Dr., 46499 Hamminkeln (DE)

(56) Entgegenhaltungen:
- EP-A- 0 374 615
- EP-A- 0 588 225
- EP-A- 0 795 533
- EP-A- 0 811 424
- EP-A- 0 823 282

## Beschreibung

Die vorliegende Erfindung betrifft ein Verfahren zur Herstellung von Aldehyden durch Umsetzung von olefinisch ungesättigten Verbindungen mit Wasserstoff und Kohlenmonoxid in homogener Phase und in Gegenwart eines metallorganische Komplexverbindungen sowie Liganden dieser Komplexverbindungen im molaren Überschuß enthaltenden Katalysatorsystems und Abtrennung des Katalysatorsystems vom Reaktionsprodukt durch Druckfiltration an einer semipermeablenMembran aus einem aromatischen Polyamid.

Die technisch in großem Umfang durchgeführte Hydroformylierung von Olefinen erfolgt zunehmend in Gegenwart von Katalysatorsystemen auf der Basis von Rhodium-Komplexverbindungen, die tertiäre Phosphine oder Phosphite als Liganden enthalten. Da die Liganden in der Regel im Überschuß vorliegen, besteht das Katalysatorsystem aus metallorganischer Komplexverbindung und zusätzlichem reinem Ligand. Entsprechend der Löslichkeit dieser Katalysatorsysteme in organischen Medien erfolgt die Hydroformylierung in homogener Phase. Zur Abtrennung des Reaktionsproduktes und Wiedergewinnung des im Reaktionsprodukt homogen gelösten Katalysatorsystems destilliert man das Reaktionsprodukt im allgemeinen aus dem Reaktionsgemisch ab. Dies ist aber wegen der thermischen Empfindlichkeit der gebildeten Aldehyde nur bei der Hydroformylierung von niederen Olefinen mit bis zu etwa 8 Kohlenstoffatomen im Molekül möglich. Bei der Hydroformylierung langkettiger Olefine oder olefinischer Verbindungen mit funktionellen Gruppen werden thermisch empfindliche Produkte oder Produkte mit hohem Siedepunkt gebildet, die sich destillativ nicht mehr zufriedenstellend vom Katalysator abtrennen lassen: Die thermische Belastung des Destillationsgutes führt durch Dickölbildung zu erheblichen Verlusten an Wertprodukt sowie durch Zersetzung der Komplexverbindungen zu Verlusten an Katalysator. Hierdurch wird die wirtschaftliche Attraktivität des Verfahrens entscheidend verringert.

Um die Abtrennung des Katalysatorsystems auf thermischem Weg zu vermeiden, wurden verschiedene Verfahren entwickelt.

Aus EP-A-0 374 615 ist es bekannt, daß sich metallorganische Komplexverbindungen aus organischen Lösungsmitteln unter Einsatz selektiver semipermeabler Polyaramid-Trennmembranen unversehrt, d.h. ohne Abbau der katalytisch aktiven Metallverbindung, abtrennen und zurückgewinnen lassen. Als treibende Kraft für den Trennvorgang kann hierbei sowohl eine Druckdifferenz (Druckfiltration) als auch eine Konzentrationsdifferenz (Dialyse) dienen. Das Verfahren eignet sich besonders zur Abtrennung von metallorganischen Komplexverbindungen und/oder Metallcarbonylen mit Phosphor(III)verbindungen als Liganden aus organischen Lösungen, in denen sie zuvor Anwendung als homogene Katalysatoren gefunden haben. Als Rhodiumkomplexverbindungen, die für die homogene Hydroformylierung von Olefinen eingesetzt werden können, sind in EP-A-0 374 615 HRhCO[P(C₆H₅)₃]₃, RhCl[P(C₆H₅)₃]₃ und solche Verbindungen genannt, die als Liganden Alkyl- oder Arylammoniumsalze sulfonierter oder carboxylierter Triarylphosphine der allgemeinen Formel 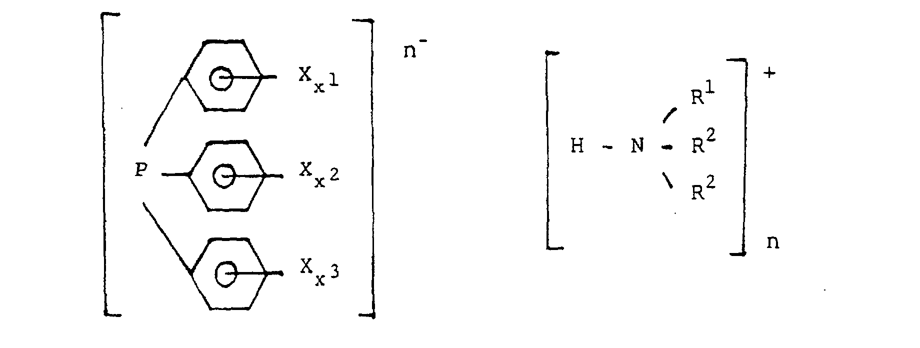 enthalten, wobei X einen Sulfonatrest (SO₃⁻) oder Carboxylatrest (COO⁻) bedeutet, x¹, x² und x³ 0 oder 1 sind, R¹ und R² jeweils gleiche oder verschiedene C₄ - C₁₂-Alkylreste, C₆ - C₁₂-Arylreste oder C₆ - C₁₂-Cycloalkylreste bedeuten und R¹ zusätzlich auch für Wasserstoff stehen kann.

Bei der zweistufigen Membranabtrennung eines Rhodium und das Triisooctylammoniumsalz von Tris(m-sulfophenyl) -phosphin enthaltenden Katalysatorsystems vom Rohprodukt der Dicyclopentadien-Hydroformylierung werden gemäß EP-A-0 374 615 99,5 % des Rhodiums und 94,4 % der Phosphor-(III) verbindung zurückgehalten. 5,6 % der Phosphor-(III) verbindung verbleiben somit im organischen Hydroformylierungsprodukt und können hieraus nur durch aufwendige Maßnahmen, wie eine komplizierte Destillation unter größeren Produktverlusten, entfernt werden. Die Flußleistung im stationären Endzustand der Membranfiltration liegt lediglich bei 5 bzw. 10 l/m²h in der ersten bzw. zweiten Membranfiltrationsstufe.

EP-823 282-A beschreibt ein Verfahren zur Herstellung von Aldehyden durch Hydroformylierung von olefinisch ungesättigten Verbindungen mit Wasserstoff und Kohlenmonoxid in homogener Phase in Gegenwart einer Rhodium-Komplexverbindung sowie aromatischer Phosphine in molarem Überschuß und die Abtrennung des Katalysatorsystems vom Reaktionsgemisch der Hydroformylierung durch Druckfiltration an einer semipermeablen Membran aus aromatischen Polyamiden.

Das aus EP 823 282-A bekannte Verfahren ist dadurch gekennzeichnet, daß die Hydroformylierung bei. einem pH-Wert von 2,5 bis 4,3 unter Verwendung eines molaren Phosphin : Rhodium-Verhältnisses von mindestens 60 und bei einer Rhodium-Konzentration von mindestens 10 Gew.-ppm, bezogen auf die eingesetzte olefinisch ungesättigte Verbindung durchgeführt wird. Als aromatische Phosphine werden aromatische Alkyl- und/oder Arylammoniumsalze sulfonierter oder carboxylierter Triarylphosphine verwendet.

EP-811 424 offenbart ein Katalysatorsystem auf Basis von Rhodium-Komplexverbindungen mit Diphosphinliganden, ein Verfahren zu ihrer Herstellung sowie die Abtrennung des Produktes von dem Katalysatorsystem durch Membranfiltration. Als Liganden werden Alkyl- oder Arylammmoniumsalze sulfonierter, carboxylierter oder phosphonierter aromatischer Diphosphine verwendet.

Es bestand daher die Aufgabe, ein Verfahren zur Hydroformylierung von olefinisch ungesättigten Verbindungen in homogener Phase zur Verfügung zu stellen, das hohe Aktivitäts- und Selektivitätswerte liefert und gleichzeitig eine verbesserte Abtrennung des gesamten Katalysatorsystems ermöglicht.

Gelöst wird diese Aufgabe durch ein Verfahren zur Herstellung von Aldehyden durch Hydroformylierung von olefinisch ungesättigten Verbindungen mit Wasserstoff und Kohlenmonoxid in homogener Phase in Gegenwart eines metallorganische Komplexverbindungen sowie Liganden dieser Komplexverbindungen im molaren Überschuß enthaltenden Katalysatorsysstems und Abtrennung des Katalysatorsystems von der Reaktionsmischung der Hydroformylierung durch Druckfiltration an einer semipermeablen Membran aus einem aromatischen Polyamid, dadurch gekennzeichnet, daß das Molmassenverhältnis der im molaren Überschuß vorliegenden Liganden zu den hergestellten Aldehyden 9 - 30, bevorzugt 10 - 25 und insbesondere 10 - 15, beträgt, wobei die Liganden keine Alkyl- oder Arlyammoniumsalze sulfonierter, carboxylierter oder phosphonierter aromatischer Diphosphine darstellen.

Nach dem neuen Prozeß gelingt es, die metallorganischen Komplexverbindungen und die im Überschuß vorliegenden Liganden dieser Komplexe unverändert, d.h. ohne daß sie zersetzt werden oder eine andere Umwandlung erfahren, nahezu verlustfrei wiederzugewinnen.

Unter metallorganischen Komplexverbindungen im Sinne der vorliegenden Erfindung versteht man Verbindungen, in denen Kohlenstoffatome organischer Gruppen an Metallatome gebunden sind. Zu den Metallen zählen auch die sogenannten Halbmetalle, wie Bor und Silicium. Als metallorganische Komplexverbindungen werden erfindungsgemäß auch solche, in einem organischen Lösungsmittel lösliche Verbindungen bezeichnet, in denen die Bindung zwischen Metall und Kohlenstoff über Stickstoff, Sauerstoff oder Schwefel erfolgt.

Das Metall der metallorganischen Komplexverbindungen ist vorzugsweise ein Element der Gruppen IVA, VA, VIA, VIIA, VIIIA oder IB des Periodensystems der Elemente, und insbesondere Mangan, Eisen, Kobalt, Nickel, Palladium, Platin, Ruthenium, Rhodium oder Iridium.

Die metallorganischen Komplexverbindungen enthalten neben den Metallen Liganden wie CO, Wasserstoff, Amine, Phosphine, Phosphite, Acetat, Benzonitril, Acetylacetonate, Dimethylglyoxime, π-Olefine wie 1,5-Cyclooctadien oder π-Aromaten wie Cyclopentadienyl.

Bei den im Überschuß im Katalysatorsystem vorliegenden Liganden handelt es sich bevorzugt um monodentale Liganden, wobei das molare Verhältnis von monodentalen Liganden zu metallorganischer Komplexverbindung in der Hydroformylierung mindestens 50, bevorzugt 60 - 120 und insbesondere 80 - 100 beträgt.

Besonders geeignet als monodentale Liganden sind aromatische Phosphine und dabei insbesondere Alkyl- und/oder Arylammoniumsalze sulfonierter oder carboxylierter Triarylphosphine. Insbesondere wird das Distearylammoniumsalz des Triphenylphosphintrisulfonats eingesetzt.

Ferner haben sich sulfonierte Pyridine, Chinoline, 2,2'-Bipyridine, Porphyrine und Pyridylphosphine, Chinin, Glyoxim, sulfonierte Phosphite sowie alkyl- und arylsubstituierte Acetylacetonate, Salicylate und Mandelate als im Überschuß vorliegende Liganden bewährt.

Die Umsetzung des Olefins mit Kohlenmonoxid und Wasserstoff erfolgt bei einer Temperatur von 100 bis 140°C, bevorzugt 120 bis 130°C und unter einem Druck von 0,5 bis 27 MPa, vorzugsweise 20 bis 25 MPa. Die Zusammensetzung des Synthesegases, d.h. das Volumenverhältnis von Koh-lenmonoxid und Wasserstoff kann sich über weite Bereiche erstrecken und z.B. zwischen 1 : 10 bis 10 : 1 variiert werden. Im allgemeinen setzt man Gasgemische ein, in denen das Volumenverhältnis von Kohlenmonoxid und Wasserstoff etwa 1 : 1 ist oder von diesem Wert nur wenig abweicht.

Im erfindungsgemäßen Verfahren werden olefinisch ungesättigte Verbindungen mit 2 bis 30 Kohlenstoffatomen umgesetzt, die eine oder mehrere Doppelbindungen besitzen können. Geeignet sind substituierte oder unsubstituierte Alkene mit 6 bis 30 Kohlenstoffatomen, substituierte oder unsubstituierte Diene mit 4 bis 10 Kohlenstoffatomen, substituierte oder unsubstituierte Cycloalkene oder Dicycloalkene mit 5 bis 12 Kohlenstoffatomen im Ringsystem, Ester einer ungesättigten Carbonsäure mit 3 bis 20 Kohlenstoffatomen und eines aliphatischen Alkohols mit 1 bis 18 Kohlenstoffatomen, Ester einer gesättigten Carbonsäure mit 2 bis 20 Kohlenstoffatomen und eines ungesättigten Alkohols mit 2 bis 18 Kohlenstoffatomen, ungesättigte Alkohle oder Ether mit jeweils 3 bis 20 Kohlenstoffatomen oder araliphatische Olefine mit 8 bis 20 Kohlenstoffatomen.

Bei den substituierten oder unsubstituierten Alkenen mit 6 bis 30 Kohlenstoffatomen kann es sich um geradkettige oder verzweigte Alkene mit endständiger oder innenständiger Lage der Doppelbindung handeln. Bevorzugt sind geradkettige Olefine mit 6 bis 18 Kohlenstoffatomen wie n-Hexen-1, n-Hepten-1, n-Octen-1, n-Nonen-1, n-Decen-1, n-Undecen-1, n-Dodecen-1, n-Octadecen-1 und acyclische Terpene. Geeignet sind auch verzweigte Alkene wie Diisobutylen (2,4,4-Trimethylpenten-1), Tripropylen, Tetrapropylen und Dimersol (Dibutylen).

Bevorzugte Beispiele für unsubstituierte Diene mit 4 bis 10 Kohlenstoffatomen sind 1,3-Butadien, 1,5-Hexadien und 1,9-Decadien.

Beispiele für substituierte und unsubstituierte Cycloalkene oder Dicycloalkene mit 5 bis 12 Kohlenstoffatomen im Ringsystem sind Cyclohexen, Cycloocten, Cyclooctadien, Dicyclopentadien und cyclische Terpene wie Limonen, Pinen, Camphoren und Bisabolen.

Beispielhaft für araliphatische Olefine mit 8 bis 20 Kohlenstoffatomen steht Styrol.

Als Beispiele für Ester einer ungesättigten Carbonsäure mit 3 bis 20 Kohlenstoffatomen und eines aliphatischen Alkohols mit 1 bis 18 Kohlenstoffatomen seien die Acrylsäureester und die Methacrylsäureester mit 1 - 18 Kohlenstoffatomen in der Alkohol-Komponente genannt.

Zu den Estern einer gesättigten Carbonsäure mit 2 - 20 Kohlenstoffatomen und eines ungesättigten Alkohols mit 2 - 18 Kohlenstoffatomen gehören die Vinyl- und Allylester mit 2 - 20 Kohlenstoffatomen in der Carbonsäurekomponente wie z.B. Vinylacetat.

Zu den ungesättigten Alkoholen und Ethern zählen beispielsweise die Allylalkohole und Vinylether.

Gegebenenfalls führt man das erfindungsgemäße Verfahren in Anwesenheit eines organischen Lösungsmittels durch, welches unter den Hydroformylierungsbedingungen inert ist und zudem die Membran in der Membranfiltrationsstufe nicht angreift. Geeignete Lösungsmittel sind aromatische Kohlenwasserstoffe, z.B. Toluol, ortho-Xylol, meta-Xylol, para-Xylol, Gemische isomerer Xylole, Ethylbenzol, Mesitylen, Gemische dieser Verbindungen oder aliphatische Kohlenwasserstoffe. Aber auch polarere Lösungsmittel, wie Acetophenon, Tetrahydrofuran, Sulfinol, Glykole oder Polyglykole, können verwendet werden. Die Hydroformylierungsreaktion kann jedoch auch ohne Zusatz eines organischen Lösungsmittels erfolgen, wobei in diesem Fall die olefinische Ausgangsverbindung und das gebildete Hydroformylierungsprodukt als Lösungsmittel wirken. Aufgrund der gewöhnlich höheren Viskosität eines solchen Reaktionsgemisches werden bei der Membranfiltration dann aber in der Regel nur geringere Flußleistungen erreicht.

Die Bildung des Katalysatorsystems aus dem Metall oder einer Metallverbindung und den Liganden erfolgt entweder in einem der Hydroformylierung vorgeschalteten Schritt, der sogenannten Präformierung, oder aber, insbesondere bei kontinuierlicher Arbeitsweise, in-situ während der Hydroformylierungsreaktion. Beide Varianten sind in der deutschen Patentanmeldung mit dem Aktenzeichen 196 19 527.6 beschrieben.

Die Herstellung der Aldehyde durch Umsetzung der in flüssiger und gasförmiger Phase vorliegenden Reaktionspartner erfolgt in konventionellen Reaktoren und kann sowohl kontinuierlich als auch diskontinuierlich durchgeführt werden.

Nach Beendigung der Hydroformylierung wird das Reaktionsgemisch in der Regel gekühlt, durch Entspannen von gasförmigen Bestandteilen befreit und mit einem Inertgas wie z.B. Stickstoff oder mit einer Synthesegasmischung aus CO und H₂ überlagert. Anschließend erfolgt die Auftrennung mittels Membranfiltration. Das Reaktionsgemisch kann jedoch auch ohne Kühlung der Membranfiltration zugeführt werden.

In dem zur Membranfiltration eingesetzten Reaktionsgemisch der Hydroformylierung beträgt die Konzentration des im Überschuß vorliegenden Liganden der metallorganischen Komplexverbindungen 2,5 - 25, bevorzugt 5 - 15 Gew.-%, bezogen auf das zur Membranfiltration eingesetzte Reaktionsgemisch.

Die Konzentration der metallorganischen Komplexverbindungen in dem zur Membranfiltration eingesetzten Reaktionsgemisch der Hydroformylierung beträgt 2 - 400 Gew.-ppm., bevorzugt 10 - 300 Gew.-ppm, insbesondere 50 - 150 Gew.-ppm, bezogen auf das zur Membranfiltration eingesetzte Reaktionsgemisch.

Die Membranfiltration erfolgt an einer Polyaramidmembran unter einem Druck von 0,1 - 15, bevorzugt 0,5 - 5, insbesondere 1 - 2 MPa.

Die Membranfiltration kann ein- oder mehrstufig durchgeführt werden, bevorzugt erfolgt sie mehrstufig, insbesondere zweistufig. Sie kann entweder mit parallel oder mit in Reihe geschalteten Trennstufen durchgeführt werden. Bevorzugt wird die Reihenschaltung, bei der in jeder Stufe das Retentat abgetrennt und die Permeat-Lösung der nächstfolgenden Trennstufe zugeleitet wird. Eine derartige Reihenschaltung erlaubt eine besonders effektive Nutzung des vorhandenen Systemdruckes, d.h. des Arbeitsdruckes im vorausgegangenen Verfahrensschritt.

Zu besonders guten Trennleistungen gelangt man, wenn die Gesamtretentatmenge 8 - 90, bevorzugt 10 - 70, insbesondere 20 - 40 %, bezogen auf das eingesetzte Reaktionsgemisch, beträgt und die Konzentration der abgetrennten Liganden im Retentat der Membranfiltration mindestens dreimal so groß ist, wie in dem zur Membranfiltration eingesetzten Reaktionsgemisch der Hydroformylierung.

Bei der zweistufigen Membranfiltration hat es sich ferner bewährt, daß das Verhältnis der Retentatmenge der 1. Filtrationsstufe zur Retentatmenge der 2. Filtrationsstufe etwa 1 : 1 beträgt.

Eine weitere Erhöhung der Trennleistung der Membran bei Anwendung der vorstehend beschriebenen Verfahrensvarianten erreicht man durch Erhöhung der Überströmung der Membran mit Hilfe einer Umwälzpumpe. Die lineare Strömungsgeschwindigkeit über der Membran liegt üblicherweise im Bereich von 0,1 - 10 m/sec, vorzugsweise 0,5 - 2,5 m/sec.

Die das Katalysatorsystem enthaltenden Retentate der Trennstufen können vereinigt werden und wieder in die Hydroformylierung zurückgeführt werden, gegebenenfalls unter ergänzendem Zusatz des Metalls und/oder der metallorganischen Komplexverbindungen sowie der Liganden dieser Komplexverbindungen. Der Zusatz dieser ergänzenden Mengen kann bei zweistufiger Durchführung der Membranfiltration auch bereits zum Permeat der 1. Stufe vor dessen Zuleitung zur 2. Membranfiltrationsstufe erfolgen.

Auf diese Weise wird ein verbessertes Trennergebnis erreicht und ein mehrfacher Wiedereinsatz des Katalysatorsystems in der Hydroformylierung ermöglicht, ohne daß nennenswerte Einbußen hinsichtlich Aktivität und Selektivität des Katalysatorsystems auftreten.

Wird das erfindungsgemäße Verfahren in Gegenwart eines Lösungsmittels durchgeführt, so kann eine besonders hohe Gesamteffizienz sowohl des Hydroformylierungs- als auch des Membrantrennschrittes erreicht werden, wenn man die Hydroformylierungsstufe mit wenig Lösungsmittel zur Erzielung eines möglichst hohen Umsatzes, die Membranstufe jedoch mit viel Lösungsmittel zur Absenkung der Viskosität betreibt. In der Hydroformylierungsstufe hat sich ein Lösungsmittelanteil von 5 - 25 Gew.-%, bevorzugt 7 - 13 Gew.%, bezogen auf das gesamte mit Lösungsmittel verdünnte Reaktionsgemisch, bewährt, im Membranfiltrationsschritt sind dagegen 30 - 70 Gew.-%, bevorzugt 40 - 60 Gew.-% Lösungsmittel, bezogen auf das gesamte mit Lösungsmittel verdünnte Reaktionsgemisch, bevorzugt. Diesen höheren Lösungsmittelanteil in der zur Membranfiltration eingesetzten Reaktionsmischung erreicht man, indem man aus den vereinigten Permeaten der Trennstufen das organische Lösungsmittel destillativ abtrennt und vor die Membranfiltration zurückführt. Dort wird es wieder dem aufzutrennenden Reaktionsgemisch der Hydroformylierung zugesetzt. Hierdurch wird eine entsprechende Verdünnung erreicht, die zur Erzielung hoher Flußleistungen dient.

Die erfindungsgemäß eingesetzten Membranen bestehen aus einem aromatischen Polyamid, auch Polyaramid genannt. Man erhält die Polyaramide durch Polykondensation aus aromatischen Dicarbonsäuren bzw. Dicarbonsäurederivaten und aromatischen Diaminen in einem dipolar aprotischen Lösungsmittel. Als Carbonsäurekomponente kommen z.B. Terephthalsäure, 4,4'-Diphenyldicarbonsäure, 4,4'-Diphenyletherdicarbonsäure, 4,4'-Diphenylsulfondicarbonsäure oder 2,6-Naphthalindicarbonsäure in Betracht. Als Diaminkomponente sind p-Phenylendiamin, 3,3'-Dimethoxybenzidin, 3,3'-Dichlorbenzidin, 3,3'-Dimethylbenzidin, 4,4'-Diaminodiphenylmethan, 2,2-Bis-(4-aminophenyl)propan oder 1,4-Bis(4-aminophenoxy)benzol geeignet.

Besondere Bedeutung haben Membranen aus solchen Polyaramiden, die neben einer Carbonsäurekomponente verschiedene Diamine als Monomere enthalten. So haben sich z.B. Polyaramide bewährt, die aus Terephthalsäure, p-Phenylendiamin, 1,4-Bis(4-aminophenoxy)benzol und 3,3'-Dimethylbenzidin aufgebaut sind. In den Polymeren können die Amine statistisch verteilt sein. Die Polyamide können aber auch die Struktur von Blockcopolymerisaten besitzen.

Das mittlere Molekulargewicht der Polyaramide kann sich über einen weiten Bereich erstrecken. Üblicherweise beträgt es 5.000 bis 200.000. Bevorzugt werden Polyaramide mit einer Molmasse von 10.000 bis 50.000.

Zur Herstellung der erfindungsgemäßen Membranen hat sich ein Verfahren bewährt, das in der deutschen Patentanmeldung P 38 02 030 beschrieben ist. Die hier offenbarten Membranen bestehen aus einem Copolyamid, das aus drei unterschiedlichen Diaminen und einer Dicarbonsäure aufgebaut ist. Eine Lösung dieses Copolyamids in einem aprotischen, polaren Lösungsmittel vom Amidtyp, z.B. N-Methyl-2-pyrrolidon, wird auf eine plane Unterlage als flüssige Schicht ausgebreitet. Diese flüssige Schicht trägt man in die Fällflüssigkeit, insbesondere Wasser, ein, die mit dem Lösungsmittel der Lösung mischbar ist, das Polymerisat jedoch als Membran abscheidet. Man läßt die Fällflüssigkeit solange auf die ausgefällte Membran einwirken, bis das Lösungsmittel vollständig durch die Fällflüssigkeit ersetzt ist. Sofern erforderlich, kann die Membran noch einer Wärmebehandlung unterzogen werden. Anschließend wird die Membran, gegebenenfalls nach vorheriger Behandlung mit Glycerin, getrocknet.

Die nach dem vorstehend beschriebenen Verfahren hergestellten Membranen sind integral asymmetrisch und sind dem Fachmann prinzipiell bekannt. Die Membranen besitzen eine sehr dünne, trennaktive Schicht, deren Dicke 0,05 bis 5 µ beträgt und eine poröse Stützstruktur. Die Dicke der aus trennaktiver Schicht und Stützstruktur bestehenden Membran kann 10 bis 400 µ betragen, sie liegt vorzugsweise im Bereich von 50 bis 200 µ.

Die Form der Membran kann beliebig gewählt werden. Sie kann als Scheibe und insbesondere als Hohlfaser oder Kapillare ausgebildet sein, aber auch jede andere, für die vorgesehene Verwendung geeignete Gestalt haben. Maßgebend ist die Erzielung einer möglichst hohen Stabilität und überdies einer größtmöglichen Oberfläche je Volumeneinheit, um einen zufriedenstellenden Durchsatz zu erreichen.

Es empfiehlt sich, die Membran vor dem Einsatz vorzubehandeln. Im einfachsten Fall taucht man sie in die zu trennende Lösung. Aber auch andere Konditionierungsverfahren sind möglich. Die zu Lagerungszwecken mit Glycerin getränkte Membran wird zunächst mit Wasser gewaschen und anschließend für 10 Minuten in 80 - 100° heißem Wasser belassen. Anschließend ersetzt man das Wasser z.B. durch i-Propanol, indem man die Membran in i-Propanol einlegt und den Alkohol mehrfach erneuert. Danach wird das i-Propanol in gleicher Weise durch das Reaktionsgemisch der Hydroformylierung ersetzt, in dem die abzutrennenden metallorganischen Komplexverbindungen sowie deren Liganden gelöst sind. Zur Erzielung einer optimalen Trennleistung hat es sich ferner bewährt, die Membran unter Arbeitsbedingungen eine gewisse Zeit einlaufen zu lassen, d.h. die Membranfiltration unter Verwendung des Reaktionsgemisches der Hydroformylierung durchzuführen, die erhaltenen Retentate und Permeate jedoch wieder zu vereinigen und in das Reaktionsgemisch der Hydroformylierung vor der Membranfiltration zurückzuführen. Durch diese sogenannte Druckkonditionierung schließen sich weitere Poren der Membran, wodurch die Trennleistung der Membran steigt. Art und Methode der Konditionierung der Membran bestimmen die beim erfindungsgemäßen Verfahren einzuhaltenden Arbeitsbedingungen.

### Beispiele

Nachfolgend wird die Herstellung einer Membran der Art beschrieben, wie sie nach dem erfindungsgemäßen Verfahren eingesetzt werden kann.

### Herstellung der Membran

Das Polyaramid wird durch Kondensation von
97-99 mol% Terephthalsäuredichlorid
25 mol% p-Phenylendiamin
25 mol% 1,4-Bis(4-aminophenoxy)benzol
50 mol% 3,3'-Dimethylbenzidin
in N-Methylpyrrolidon als Lösungsmittel hergestellt. Terephthalsäuredichlorid setzt man in einer solchen Menge ein, daß das Polyaramid einen Staudinger-Index von 200 bis 300 ml/g hat. Die Lösungsmittelmenge wird so bemessen, daß eine etwa 7 Gew.-% Polykondensat enthaltende Lösung entsteht. Nachdem die Kondensation erfolgt ist, wird der locker an das Lösungsmittel gebundene Chlorwasserstoff durch Zugabe von 100 mol% CaO neutralisiert. Anschließend löst man in dem Reaktionsgemisch unter Rühren 5 Gew.-% (bezogen auf die Polymerlösung) wasserfreies Calciumchlorid. Die Lösung wird gelinde erwärmt, filtriert und entgast. Sie kann unmittelbar zur Membranherstellung verwendet werden.

Es ist möglich, die Membran trägerfrei oder auf einem Polyestervlies als Träger herzustellen. Im folgenden wird die Herstellung einer trägerfreien Membran beschrieben.

Die leicht erwärmte Polyaramidlösung wird mit einer Rakel auf einer Glasplatte zu einem gleichmäßigen Film von etwa 150 µ ausgezogen und in ein Wasserbad von 2°C eingetaucht. Nach etwa 20 min zieht man die Membran von der Glasplatte ab und legt sie 5 min in 100°C heißes Wasser. Darauf gibt man die Membran in i-Propanol, um die Porenflüssigkeit Wasser gegen den Alkohol auszutauschen. Anschließend wird die Membran mit Toluol gewaschen, nach dieser Behandlung ist sie zur Durchführung der Trennungen geeignet. Bei allen Operationen ist darauf zu achten, daß die Membran nicht austrocknet.

### Beispiele 2 - 6 und Vergleichsbeispiele 1, 7 und 8

Hydroformylierung von Dicyclopentadien (DCP) unter Verwendung von Katalysatorsystemen, die Rhodium und verschiedene Ammoniumsalze des Triphenylphosphintrisulfonats (TPPTS) enthalten:

### a) Herstellung des Distearylammoniumsalzes des TPPTS

253 g einer Na-TPPTS-Lösung werden unter Stickstoff in einem Rührkolben vorgelegt und auf 65°C erwärmt. Anschließend fügt man eine Lösung von 250,3 g Distearylamin in 595 g Toluol hinzu. Innerhalb von 60 min gibt man unter Rühren 90 ml 20%ige Schwefelsäure zu, bis ein pH-Wert von 2,6 erreicht ist, und läßt 2,5 h nachreagieren. Zur besseren Phasentrennung werden 170 g Isopropanol zugegeben. Nach 15 min werden 1037,5 g einer organischen Phase, die das Distearylammoniumsalz des TPPTS mit 0,33 mol TPPTS pro mol Amin enthält, abgetrennt. Die organische Phase enthält 126 mmol Phosphor(III)/kg.
Andere Ammoniumsalze des TPPTS (Beispiele 2 - 7 und Vergleichsversuch 1) werden analog zu obiger Vorschrift dargestellt. Die in Beispiel 3 sowie den Vergleichsbeispielen 7 und 8 eingesetzten Jeffaminen stellen Handelprodukte der Texaco Chemical Corporation dar und besitzen folgende Struktur:

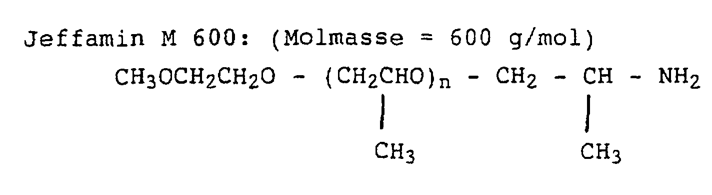

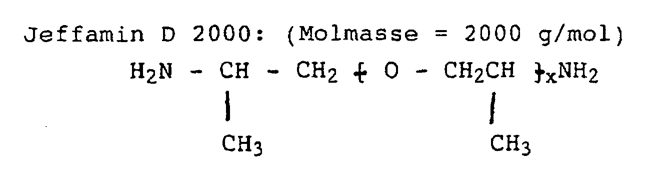

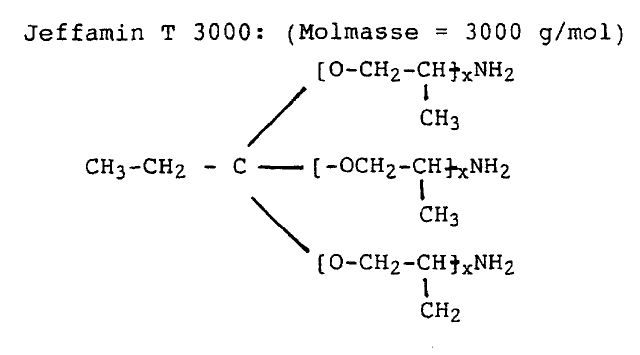

### b) Diskontinuierliche Hydroformylierung von Dicyclopentadien unter Verwendung des Distearylammoniumsalzes des TPPTS.

Ein 2,15 l Rührautoklav wird mit Stickstoff gespült. In einer Glasvorlage mit Stickstoffüberlagerung werden 212,8 g der Ligandlösung aus a) sowie 0,29 mmol Rhodium in Form eines 2-Ethylhexanoatsalzes gelöst (60 Gew.-ppm Rh; P/Rh-Verhältnis: 100) und 500 g Toluol unter Stickstoff in den Autoklaven überführt. Danach wird unter Rühren durch Zufuhr von Synthesegas ein Druck von 27 MPa eingestellt. Nach Erreichen einer Reaktionstemperatur von 130°C läßt man zwei Stunden präformieren. Danach werden innerhalb von 1 Stunde 500 g Dicyclopentadien in den Autoklaven gepumpt. Durch Kühlung mit einem Luftgebläse wird die Temperatur von 130°C gehalten. Nach Ende der Dicyclopentadien-Zufuhr läßt man noch 3 Stunden nachreagieren. Anschließend wird der Autoklav auf Raumtemperatur abgekühlt und entspannt. Der Autoklaveninhalt wird dann mit Restdruck in einen 2 l Dreihalskolben mit Tauchstutzen überführt und gewogen. Aus der Gewichtszunahme errechnet sich der Dicyclopentadienumsatz.

Die Hydroformylierung des Dicyclopentadiens unter Verwendung der Ammoniumsalze des TPPTS gemäß den Beispielen 3-6 und der Vergleichsversuche 1, 7 und 8 wird analog durchgeführt. Die erhaltenen Ergebnisse sind in Tabelle 1 zusammengefaßt.

### c) Einstufige Membranfiltration

Das jeweilige obige Reaktionsprodukt aus b) wird über eine Labormembranfilteranlage gegeben. Als Membran wird eine Polyaramidmembran der Firma Hoechst AG (UF-PA (PET 100)) verwendet. Die Membran wird zunächst 10 min bei 80°C in Wasser getempert. Mittels einer Umwälzpumpe wird die Membran dann mit 200 l/h überströmt und ein Druck von 1 MPa eingestellt. Bei einer Betriebstemperatur von 40°C passiert die in Tabelle 1 angegebene Menge des Hydroformylierungsproduktes die Membran als Permeat. Im Permeat wird der Gehalt an Katalysatorbestandteilen bestimmt, woraus sich, bezogen auf das eingesetzte Reaktionsgemisch der Hydroformylierung, die in Tabelle 1 angegebenen Rückhaltewerte ergeben.

Aus Tabelle 1 ist zu erkennen, daß nur bei Einhaltung eines Molmassenverhältnisses [Molmasse (Ligand) : Molmasse (Aldehyd)] = 9 - 30 sowohl bei der Hydroformylierung ausgezeichnete Selektivitäten als auch bei der Membranabtrennung hervorragende Rückhaltewerte erzielt werden.

### Beispiel 9

Ein 5 l Rührautoklav mit einem Tauchstutzen für Gaseinlaß und Entnahme von Produkten sowie einem Gasauslaßventil wird sorgfältig mit Stickstoff gespült.

872 g einer toluolischen Lösung des Distearylammoniumsalzes von TPPTS mit einem Phosphor(III)-Gehalt von 138 mmol/kg sowie 120 mg Rhodium in Form von Rh-2-Ethylhexanoat werden aus einer Vorlage mit Stickstoffüberdruck in den Autoklaven überführt.

Anschließend wird der Katalysator 2 Stunden bei 27 MPa und 125°C präformiert. Dann werden innerhalb von 1 Stunde 1500 g Propylen aus einer Vorlage über das Tauchrohr eingepumpt (80 ppm Rh, bezogen auf Propylen; molares P : Rh-Verhältnis = 100). Die Reaktionswärme wird über Kühlschlangen im Reaktor abgeführt. Man läßt noch 1 Stunde nachreagieren und abkühlen. Der Autoklav wird entspannt, in einen 3-Halskolben mit Schlenktechnik überführt und gewogen (3028 kg). Der Umsatz liegt bei 95 %, das n/i-Verhältnis bei 63/37.

Anschließend wird das Reaktionsprodukt in eine Labor-Membrananlage überführt und 2-stufig filtriert. Der Transmembrandruck liegt bei 1,5 MPa. Es wird eine UF-PA5 (PET 100) Membran der Hoechst AG eingesetzt. Es werden die in der Tabelle 2 angegebenen Rückhaltwerte und Flußleistungen erreicht.

Die Retentate der 1. und 2. Stufe werden anschließend zurückgeführt und im Autoklaven erneut mit Propylen umgesetzt. Die sehr niedrigen Verluste an Rh und Phosphor(III) werden entsprechend durch Zusatz von Rhodium-2-ethylhexanoat bzw.einer Lösung des Distearylammoniumsalzes des TPPTS ausgeglichen. Die Ergänzung erfolgt zum Permeat der 1. Stufe.

Der Katalysator wird insgesamt 10 mal rezirkuliert, ohne daß der Umsatz (90 - 95 %), die Selektivität (n/i-Verhältnis 63/37) oder die Rückhaltwerte (siehe Tabelle 2) sich wesentlich ändern. Bei den in Tabelle 2 angegebenen Werten für die Flußleistung stellt der erste Wert den Anfangswert in der jeweiligen Membranfiltrationsstufe dar, während der zweite Wert den Gleichgewichtszustand charakterisiert.

Tabelle 2 zeigt, daß im Wiedereinsatz die Flußleistung infolge einer sich aufbauenden Konzentration an Dickölen zunächst zurückgeht, sich aber auf dem niedrigeren Niveau stabilisiert, d.h. daß auch die Dicköle permeieren und so eine Trennung von Katalysatorbestandteilen und Dicköl möglich ist.

Tabelle 2 zeigt weiterhin, daß nach dem erfindungsgemäßen Verfahren erstmals Metallkomplexe und überschüssige Liganden ausgezeichnet von den Produkten einschließlich Dicköl abgetrennt und rezirkuliert werden können.

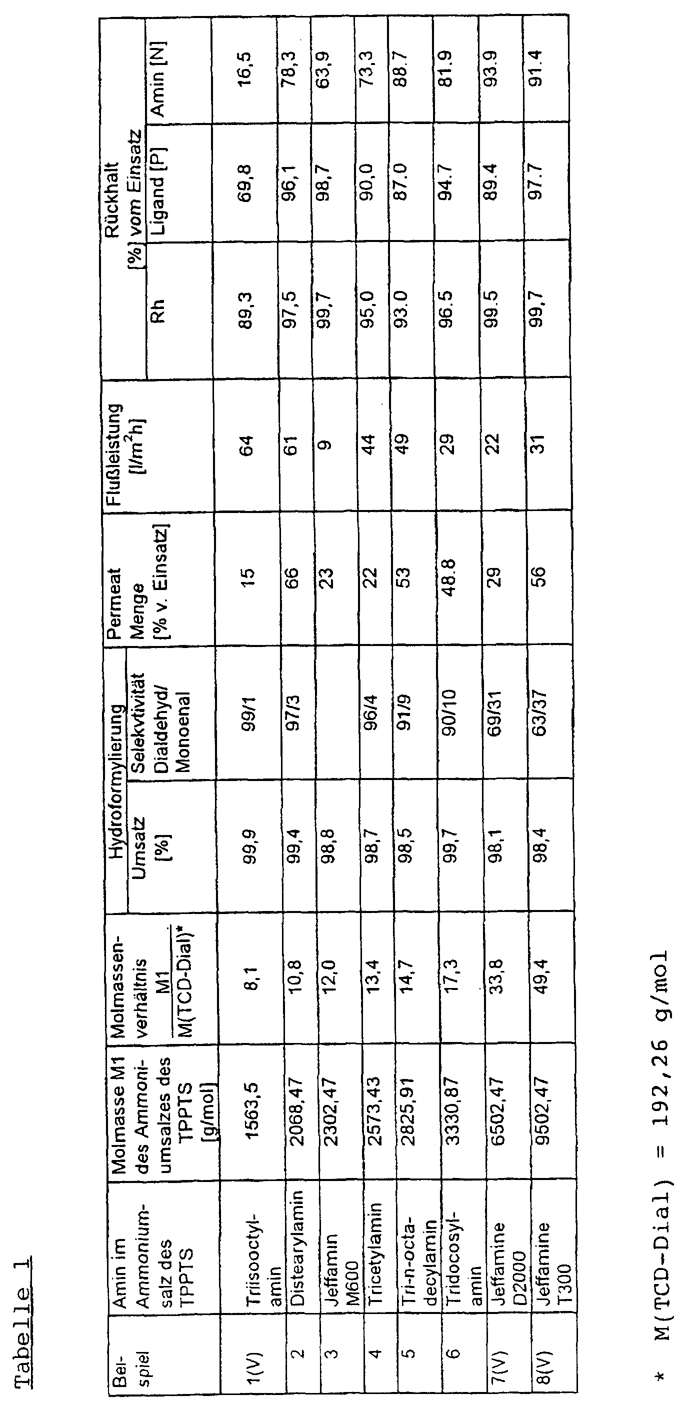

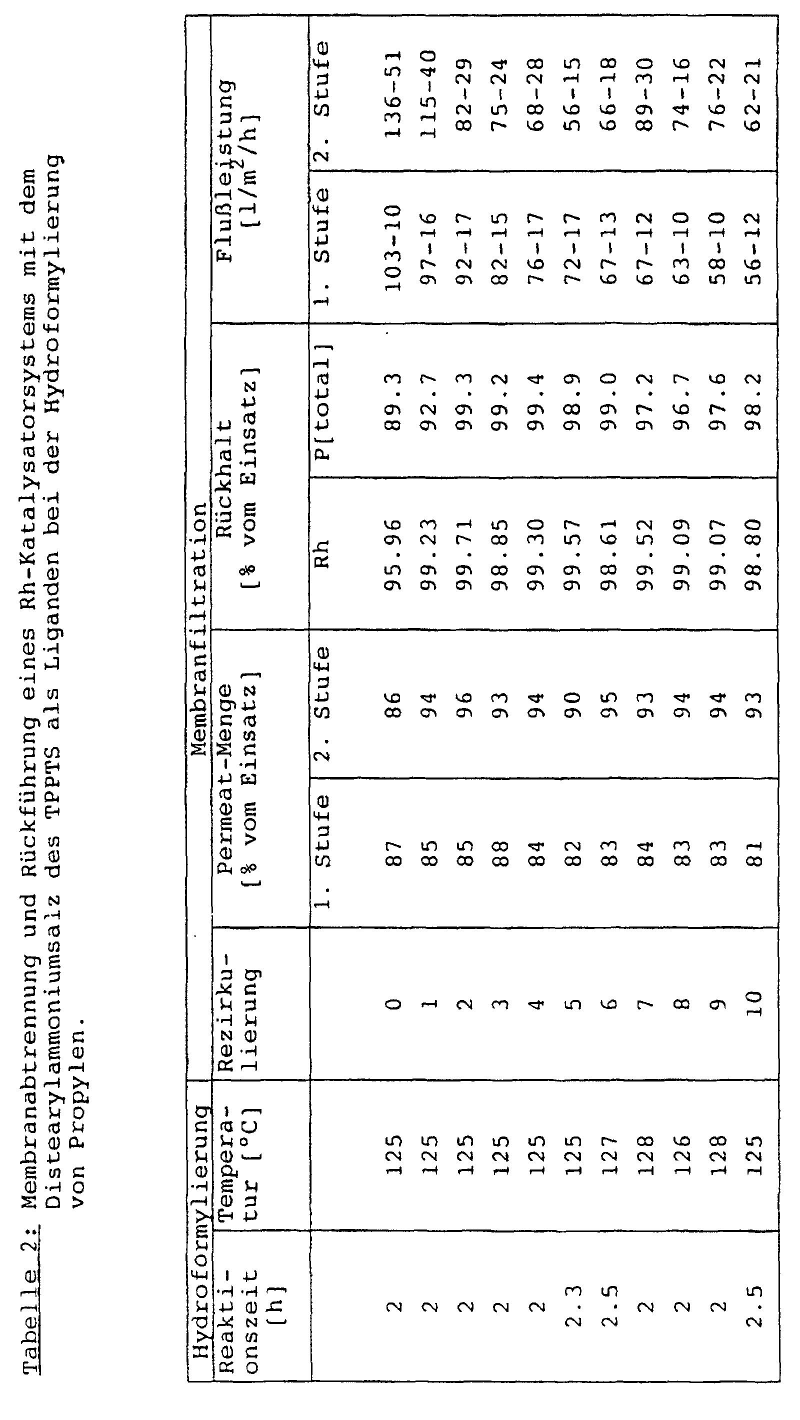

## Patentansprüche

1. Verfahren zur Herstellung von Aldehyden durch Hydroformylierung von olefinisch ungesättigten Verbindungen mit Wasserstoff und Kohlenmonoxid in homogener Phase in Gegenwart eines metallorganische Komplexverbindungen sowie Liganden dieser Komplexverbindungen im molaren Überschuß enthaltenden Katalysatorsystems und Abtrennung des Katalysatorsystems von der Reaktionsmischung der Hydroformylierung durch Druckfiltration an einer semipermeablen Membran aus einem aromatischen Polyamid, **dadurch gekennzeichnet, daß** das Molmassenverhältnis der im Überschuß vorliegenden Liganden zu den hergestellten Aldehyden 9-30, bevorzugt 10 - 25, insbesondere 10 - 15, beträgt, wobei die im Überschuß vorliegenden Liganden keine Alkyl- oder Arylammoniumsalze sulfonierter, carboxylierter oder phosphonierter aromatischer. Diphosphine oder keine Verbindung oder kein Gemisch von Verbindungen der allgemeinen Formel (I) 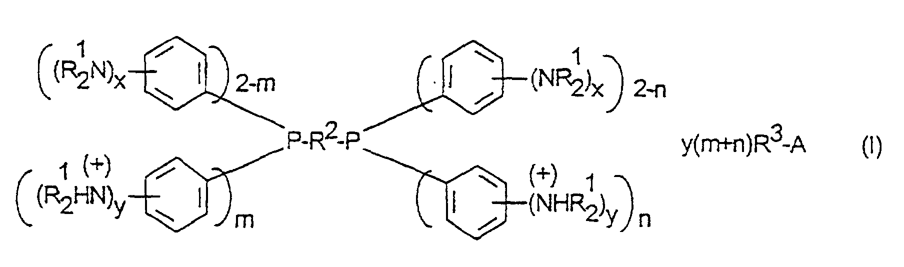 worin R¹ für H oder einen Alkylrest mit 1 bis 12 Kohlenstoffatomen, R² für einen geradkettigen Alkylenrest mit 1 bis 8 Kohlenstoffatomen, einen Sauerstoff enthaltenden Alkylenrest mit 2 bis 4 Kohlenstoffatomen, einen Rest der Formel (II) oder (III) 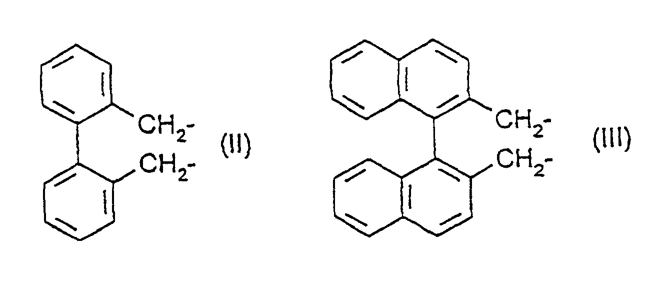 oder einen Cycloalkylenrest mit 3 bis 10 Kohlenstoffatomen, R³ für einen Alkylrest mit 1 bis 25 Kohlenstoffatomen oder einen Arylrest mit 6 bis 10 Kohlenstoffatomen steht, A einen Rest -COO⁻ oder -SO₃⁻ darstellt und x = 0, y = 1, m = 1 und n = 1 ist, oder x = 1, y = 1, m = (1 oder 2) und n = (1 oder 2) ist, oder, falls R² einen Rest der Formel (II) oder (III) darstellt, x = 1, y = 0, m = (0 oder 1) und n = (0 oder 1) ist, darstellen.

2. Verfahren nach Anspruch 1, **dadurch gekennzeichnet, daß** das Metall der metallorganischen Komplexverbindungen ein Element der Gruppen IVA, VA, VIA, VIIA, VIIIA oder IB des Periodensystems der Elemente und insbesondere Mangan, Eisen, Kobalt, Nickel, Palladium, Platin, Ruthenium, Rhodium oder Iridium ist.

3. Verfahren nach Anspruch 1 oder 2, **dadurch gekennzeichnet, daß** es sich bei den im Überschuß vorliegenden Liganden um monodentale Liganden handelt und das molare Verhältnis von monodentalen Liganden zu metallorganischer Komplexverbindung in der Hydroformylierung mindestens 50, bevorzugt 60 - 120 und insbesondere 80 - 100 beträgt.

4. Verfahren nach einem oder mehreren der Ansprüche 1-3, **dadurch gekennzeichnet, daß** es sich bei den im Überschuß vorliegenden Liganden um sulfonierte Pyridine, Chinoline, 2,2'-Bipyridine, Porphyrine und Pyridylphosphine, Chinin, Glyoxim, sulfonierte Phosphite sowie alkyl- und arylsubstituierte Acetylacetonate, Salicylate oder Mandelate handelt.

5. Verfahren nach einem oder mehreren der Ansprüche 1-4, **dadurch gekennzeichnet, daß** die Umsetzung des Olefins mit Kohlenmonoxid und Wasserstoff bei einer Temperatur von 100 bis 140°C, bevorzugt 120 bis 130°C und unter einem Druck von 0,5 bis 27 MPa, vorzugweise 20 bis 25 MPa erfolgt.

6. Verfahren nach einem oder mehreren der Ansprüche 1-5, **dadurch gekennzeichnet, daß** zur Hydroformylierung olefinisch ungesättigte Verbindungen mit 2 bis 30 Kohlenstoffatomen umgesetzt werden, die eine oder mehrere Doppelbindungen besitzen können.

7. Verfahren nach einem oder mehreren der Ansprüche 1-6, **dadurch gekennzeichnet, daß** man in Anwesenheit eines organischen Lösungsmittels arbeitet.

8. Verfahren nach einem oder mehreren der Ansprüche 1-7, **dadurch gekennzeichnet, daß** in dem zur Membranfiltration eingesetzten Reaktionsgemisch der Hydroformylierung die Konzentration der im Überschuß vorliegenden Liganden der Komplexverbindungen 2,5 - 25, bevorzugt 5 - 15 Gew.-%, bezogen auf das zur Membranfiltration eingesetzte Reaktionsgemisch beträgt.

9. Verfahren nach einem oder mehreren der Ansprüche 1-8, **dadurch gekennzeichnet, daß** die Konzentration der metallorganischen Komplexverbindungen in dem zur Membranfiltration eingesetzten Reaktionsgemisch der Hydroformylierung 2 - 400 Gew.-ppm, bevorzugt 10-300 Gew.-ppm, insbesondere 50 - 150 Gew.-ppm, bezogen auf das zur Membranfiltration eingesetzte Reaktionsgemisch, beträgt.

10. Verfahren nach einem oder mehreren der Ansprüche 1-9, **dadurch gekennzeichnet, daß** die Membranfiltration an einer Polyaramidmembran unter einem Druck von 0,1 - 15, bevorzugt 0,5 - 5, insbesondere 1 - 2 MPa erfolgt und ein- oder mehrstufig, bevorzugt zweistufig, durchgeführt wird.

11. Verfahren nach einem oder mehreren der Ansprüche 1-10, **dadurch gekennzeichnet, daß** die Membranfiltration mit in Reihe geschalteten Trennstufen durchgeführt wird.

12. Verfahren nach einem oder mehreren der Ansprüche 1-11, **dadurch gekennzeichnet, daß** die Gesamtretentatmenge der Membranfiltration 8 - 90, bevorzugt 10-70 % und insbesondere 20 - 40 %, bezogen auf das eingesetzte Reaktionsgemisch der Hydroformylierung, beträgt und die Konzentration der abgetrennten Liganden der Komplexverbindungen im Retentat mindestens dreimal so groß ist wie in dem zur Membranfiltration eingesetzten Reaktionsgemisch der Hydroformylierung.

13. Verfahren nach einem oder mehreren der Ansprüche 1-12, **dadurch gekennzeichnet, daß** bei der zweistufigen Membranfiltration das Verhältnis der Retentatmenge der 1. Filtrationsstufe zur Retentatmenge der 2. Stufe etwa 1 : 1 beträgt.

14. Verfahren nach einem oder mehreren der Ansprüche 1-13, **dadurch gekennzeichnet, daß** die das Katalysatorsystem enthaltenden Retentate der Trennstufen der Membranfiltration wieder in die Hydroformylierung zurückgeführt werden, gegebenenfalls unter ergänzendem Zusatz des Metalls und/oder der metallorganischen Komplexverbindungen sowie der Liganden dieser Komplexverbindungen.

15. Verfahren nach Anspruch 14, **dadurch gekennzeichnet, daß** bei zweistufiger Durchführung der Membranfiltration der ergänzende Zusatz des Metalls und/oder der metallorganischen Komplexverbindungen und der Liganden dieser Komplexverbindungen bereits zum Permeat der 1. Filtrationsstufe vor dessen Zuleitung zur 2. Filtrationsstufe erfolgt,

16. Verfahren nach einem oder mehreren der Ansprüche 1-15, **dadurch gekennzeichnet, daß** aus den vereinigten Permeaten der Trennstufen der Membranfiltration das Lösungsmittel destillativ abgetrennt wird, vor die Membranfiltration zurückgeführt und dem Reaktionsgemisch der Hydroformylierung vor der Membranfiltration zugesetzt wird.

## Claims

1. A process for preparing aldehydes by hydroformylation of olefinically unsaturated compounds with hydrogen and carbon monoxide in a homogeneous phase in the presence of a catalyst system comprising organometallic complex compounds and ligands of these complex compounds in molar excess, and separating off the catalyst system from the hydroformylation reaction mixture by pressure filtration on a semipermeable membrane of an aromatic polyamide, in which the molar mass ratio of the ligands present in excess to the aldehydes prepared is 9-30, preferably 10-25, and in particular 10-15, with the ligands present in excess not being alkylammonium or arylammonium salts of sulfonated, carboxylated or phosphonated aromatic diphosphines, or no compound or no mixture of compounds of the formula (I) 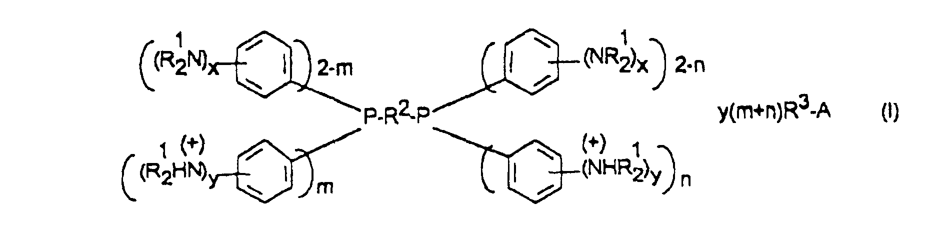 in which R¹ is H or an alkyl radical having 1 to 12 carbon atoms, R² is a staight-chain alkylene radical having 1 to 8 carbon atoms, an oxygen-containing alkylene radical having 2 to 4 carbon atoms, a radical of the formula (II) or (III) 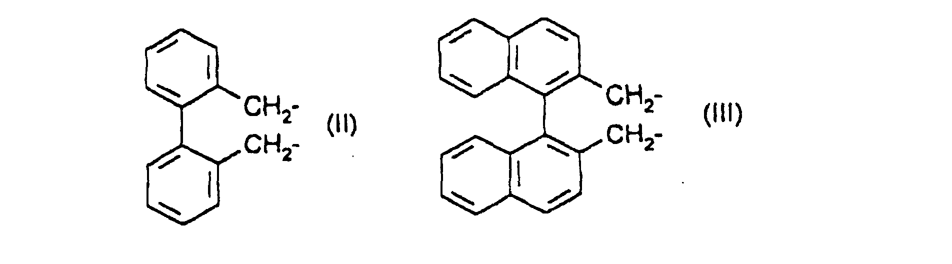 or a cycloalkylene radical having 3 to 10 carbon atoms, R³ is an alkyl radical having 1 to 25 carbon atoms or an aryl radical having 6 to 10 carbon atoms, A is a radical -COO⁻ or -SO₃⁻ and x=0, Y=1, m=1 and n=1, or x=1, y=1, m=(1 or 2) and n=(1 or 2), or, if R² is a radical of the formula (II) or (III), x=1, y=0, m=(0 or 1) and n=(0 or 1).

2. The process as claimed in claim 1, wherein the metal of the organometallic complex compounds is an element of the groups IVA, VA, VIA, VIIA, VIIIA or IB of the Periodic Table of the Elements and is, in particular, manganese, iron, cobalt, nickel, palladium, platinum, ruthenium, rhodium or iridium.

3. The process as claimed in claim 1 or 2, wherein the ligands present in excess are monodentate ligands and the molar ratio of monodentate ligands to organometallic complex compound in the hydroformylation is at least 50, preferably 60-120, and in particular 80-100.

4. The process as claimed in one or more of claims 1-3, wherein the ligands present in excess are sulfonated pyridines, quinolines, 2,2'-bipyridines, porphyrins and pyridylphosphines, quinine, glyoxime, sulfonated phosphites or alkyl- and aryl-substituted acetylacetonates, salicylates or mandeltes.

5. The process as claimed in one or more of claims 1-4, wherein the olefin is reacted with carbon monoxide and hydrogen at a temperature of 100 to 140°C, preferably 120 to 130°C and at a pressure of 0.5 to 27 MPa, preferably 20 to 25 MPa.

6. The process as claimed in one or more of claims 1-5, wherein, for the hydroformylation, olefinically unsaturated compounds having 2 to 30 carbon atoms, which can have one or more double bonds, are reacted.

7. The process as claimed in one or more of claims 1-6, wherein it is carried out in the presence of an organic solvent.

8. The process as claimed in one or more of claims 1-7, wherein, in the hydroformylation reaction mixture used for the membrane filtration, the concentration of the complex compound ligands present in excess is 2.5-25, preferaby 5-15, % by weight, based on the reaction mixture used for the membrane filtration.

9. The process as claimed in one or more of claims 1-8, wherein the concentration of the organometallic complex compounds in the hydroformylation reaction mixture used for the membrane filtration is 2-400 ppm by weight, preferably 10-300 ppm by weight, in particular 50-150 ppm by weight, based on the reaction mixture used for the membrane filtration.

10. The process as claimed in one or more of claims 1-9, wherein the membrane filtration is performed on a polyaramid membrane at a pressure of 0.1-15, preferably 0.5-5, in particular 1-2, MPa, and is carried out in a single stage or in multiple stages, preferably in two stages.

11. The process as claimed in one or more of claims 1-10, wherein the membrane filtration is carried out with series-connected separation stages.

12. The process as claimed in one or more of claims 1-11, wherein the total amount of retentate of the membrane filtration is 8-90, preferably 10-70, %, and in particular 20-40%, based on the hydroformylation reaction mixture used, and the concentration of the separated ligands of the complex compounds in the retentate is at least three times as high as in the hydroformylation reaction mixture used for the membrane filtration.

13. The process as claimed in one or more of claims 1-12, wherein, in the two-stage membrane filtration, the ratio of the amount of retentate of the 1st filtration stage to the amount of retentate of the 2nd stage is about 1:1.

14. The process as claimed in one or more of claims 1-13, wherein the membrane filtration separation stage retentates containing the catalyst system are recycled back to the hydroformylation, with or without supplementary addition of the metal and/or of the organometallic complex compounds and of the ligands of these complex compounds.

15. The process as claimed in claim 14, wherein, in the case of a two-stage membrane filtration procedure, the supplementary addition of the metal and/or of the organometallic complex compounds and of the ligands of these complex compounds is even made to the permeate of the 1st filtration stage prior to its feed to the 2nd filtration stage.

16. The process as claimed in one or more of claims 1-15, wherein the solvent is separated off from the combined permeates of the membrane filtration separation stages by distillation, recycled upstream of the membrane filtration and added to the hydroformylation reaction mixture upstream of the membrane filtration.

## Revendications

1. Procédé de production d'aldéhydes par hydroformylation de composés oléfiniquement insaturés avec l'hydrogène et le monoxyde de carbone en phase homogène, en présence d'un système catalytique contenant des complexes organométalliques ainsi que des ligands de ces complexes en excès molaire et séparation du système catalytique d'avec le mélange réactionnel d'hydroformylation par filtration sous pression sur une membrane semi-perméable constituée par un polyamide aromatique, **caractérisé en ce que** le rapport molaire des ligands présents en excès aux aldéhydes produits est 9-30, de préférence 10-25, en particulier 10-15, où les ligands présents en excès ne sont pas des sels d'alkyl- ou arylammonium de diphosphines aromatiques sulfonées, carboxylées ou phosphonées et ne sont pas un composé ou un mélange de composés de formule générale (I) 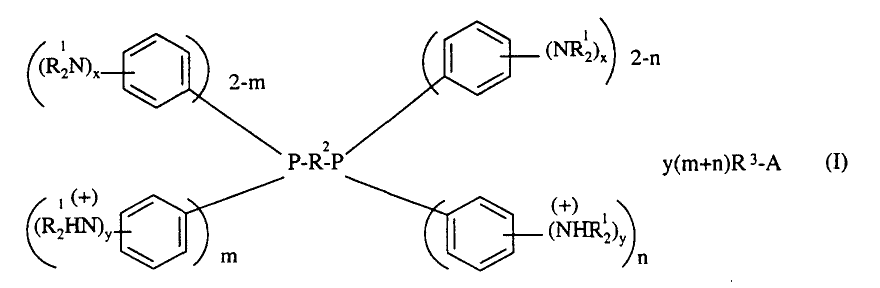 où R¹ représente H ou un reste alkyle de 1 à 12 atomes de carbone, R² représente un reste alkylène linéaire de 1 à 8 atomes de carbone, un reste alkylène contenant de l'oxygène de 2 à 4 atomes de carbone, un reste de formule (II) ou (III) 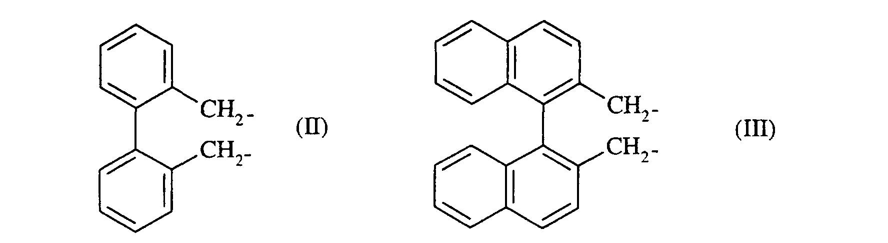 ou un reste cycloalkylène de 3 à 10 atomes de carbone, R³ représente un reste alkyle de 1 à 25 atomes de carbone ou un reste aryle de 6 à 10 atomes de carbone, A représente un reste -COO⁻ ou -SO₃⁻ et x = 0, y = 1, m = 1 et n = 1, ou bien x = 1, y = 1, m = (1 ou 2) et n = (1 ou 2), ou bien, dans le cas ou R² représente un reste de formule (II) ou (III), x = 1, y = 0, m = (0 ou 1) et n = (0 ou 1).

2. Procédé selon la revendication 1, **caractérisé en ce que** le métal des complexes organométalliques est un élément des groupes IVA, VA, VIA, VIIA, VIIIA ou IB du système périodique des éléments, et en particulier le manganèse, le fer, le cobalt, le nickel, le palladium, le platine, le ruthénium, le rhodium ou l'iridium.

3. Procédé selon la revendications 1 ou 2, **caractérisé en ce que** les ligands présents en excès sont des ligands monodentés et le rapport molaire des ligands monodentés au complexe organométallique dans l'hydroformylation est d'au moins 50, de préférence de 60-120 et en particulier de 80-100.

4. Procédé selon une ou plusieurs des revendications 1 à 3, **caractérisé en ce que** les ligands présents en excès sont des pyridines sulfonées, des quinoléines, des 2,2'-bipyridines, des porphyrines et des pyridylphosphines, la quinine, le glyoxime, des phosphites sulfonés ainsi que des acétylacétonates, salicylates ou mandélates alkyl- ou arylsubstitués.

5. Procédé selon une ou plusieurs des revendications 1 à 4, **caractérisé en ce que** la réaction de l'oléfine avec le monoxyde de carbone et l'hydrogène se déroule à une température de 100 à 140°C, de préférence de 120 à 130°C et sous une pression de 0,5 à 27 MPa, de préférence de 20 à 25 MPa.

6. Procédé selon une ou plusieurs des revendications 1 à 5, **caractérisé en ce que**, pour l'hydroformylation, on fait réagir des composés oléfiniquement insaturés à 2 à 30 atomes de carbone qui peuvent posséder une ou plusieurs doubles liaisons.

7. Procédé selon une ou plusieurs des revendications 1 à 6, **caractérisé en ce que** l'on opère en présence d'un solvant organique.

8. Procédé selon une ou plusieurs des revendications 1 à 7, **caractérisé en ce que**, dans le mélange réactionnel d'hydroformylation utilisé pour la filtration sur membrane, la concentration des ligands présents en excès des complexes est 2,5-25, de préférence 5-15 % en masse, par rapport au mélange réactionnel utilisé pour la filtration sur membrane.

9. Procédé selon une ou plusieurs des revendications 1 à 8, **caractérisé en ce que** la concentration des complexes organométalliques dans le mélange réactionnel d'hydroformylation utilisé pour la filtration sur membrane est 2-400 ppm en masse, de préférence 10-300 ppm en masse, en particulier 50-150 ppm en masse, par rapport au mélange réactionnel utilisé pour la filtration sur membrane.

10. Procédé selon une ou plusieurs des revendications 1 à 9, **caractérisé en ce que** la filtration sur membrane est réalisée sur une membrane en polyaramide sous une pression de 0,1-15, de préférence 0,5-5, en particulier 1-2 MPa et en une ou plusieurs étapes, de préférence en deux étapes.

11. Procédé selon une ou plusieurs des revendications 1 à 10, **caractérisé en ce que** la filtration sur membrane est réalisée avec des étapes de séparation branchées en série.

12. Procédé selon une ou plusieurs des revendications 1 à 11, **caractérisé en ce que** la quantité totale de rétentat de la filtration sur membrane est 8-90, de préférence 10-70 % et en particulier 20-40 %, par rapport au mélange réactionnel d'hydroformylation utilisé, et la concentration des ligands séparés des complexes dans le rétentat est au moins trois fois plus élevée que dans le mélange réactionnel d'hydroformylation utilisé pour la filtration sur membrane.

13. Procédé selon une ou plusieurs des revendications 1 à 12, **caractérisé en ce que**, dans la filtration sur membrane en deux étapes, le rapport de la quantité de rétentat de la première étape de filtration à la quantité de rétentat de la deuxième étape est d'environ 1:1.

14. Procédé selon une ou plusieurs des revendications 1 à 13, **caractérisé en ce que** les rétentats des étapes de séparation de la filtration sur membrane contenant le système catalytique sont renvoyés dans l'hydroformylation, éventuellement avec addition complémentaire du métal et/ou des complexes organométalliques ainsi que des ligands de ces complexes.

15. Procédé selon la revendication 14, **caractérisé en ce que**, dans la mise en oeuvre en deux étapes de la filtration sur membrane, l'addition complémentaire du métal et/ou des complexes organométalliques et des ligands de ces complexes a lieu déjà au perméat de la première étape de filtration avant son introduction dans la deuxième étape de filtration.

16. Procédé selon une ou. plusieurs des revendications 1 à 15, **caractérisé en ce que** le solvant est séparé par distillation des perméats réunis des étapes de séparation de la filtration sur membrane, recyclé avant la filtration sur membrane et ajouté au mélange réactionnel d'hydroformylation avant la filtration sur membrane.
